Europäisches Patentamt

European Patent Office

Office européen des brevets

(11) Publication number: **0 058 474**
**B1**

(12) **EUROPEAN PATENT SPECIFICATION**

(45) Date of publication of patent specification: **02.01.86**

(51) Int. Cl.⁴: **A 61 K 7/00,** A 61 K 7/48

(21) Application number: **82300335.5**

(22) Date of filing: **22.01.82**

(54) **Cosmetic product.**

(30) Priority: **26.01.81 IT 6709681**

(43) Date of publication of application:
**25.08.82 Bulletin 82/34**

(45) Publication of the grant of the patent:
**02.01.86 Bulletin 86/01**

(84) Designated Contracting States:
**AT BE CH DE FR GB IT LI NL SE**

(56) References cited:
**DE-A-2 225 313**
**FR-A-2 417 980**
**GB-A-1 156 812**
**GB-A-1 555 796**
**GB-A-2 072 503**
**US-A-3 098 795**
**US-A-3 211 619**
**US-A-3 856 931**

**Cosmetics, Vol. 1, pages 376-379, Second
Edition**

(73) Proprietor: **UNILEVER PLC**
**Unilever House Blackfriars P.O. Box 68
London EC4P 4BQ (GB)**
(84) **GB**

(73) Proprietor: **UNILEVER NV
Burgemeester s'Jacobplein 1 P.O. Box 760
NL-3000 DK Rotterdam (NL)**
(84) **BE CH DE FR IT LI NL SE AT**

(72) Inventor: **Caserio, Domenico
Via Astico 13
I-20131 Milan (IT)**
Inventor: **Mignini, Elio
Via Gabbro 19
I-20161 Milan (IT)**

(74) Representative: **Tonge, Robert James et al
UNILEVER PLC Patents Division P.O. Box 68
Unilever House
London EC4P 4BQ (GB)**

Courier Press, Leamington Spa, England.

# Description

The invention relates to a cosmetic stick for application to the skin, particularly for the prevention or treatment of acne or of other skin disorders.

The primary symptom of acne is a disorder in the keratinisation of the upper part of the pilosebacous follicle. The follicular ostium becomes obstructed by hyperkeratinised and cohesive horny cells to form a microcomedone. Due to the accumulation of these hyperkeratinised cells, the follicle develops into a microcyst which may evolve as an inflammatory lesion known as a papule, or a non-inflammatory lesion known as an open comedone. The external orifice of the follicle is not visible in the microcyst, but in the open comedone, it becomes distended by a mass of darkly pigmented horney cells.

As acne develops, the follicular epithelium may break-up and cause an eruption into the dermis of keratin and sebum. The sebum contains free fatty acids derived primarily from the lytic effect of bacterial lipases (especially from *Corynebacterium acne*) on sebum triglycerides. Inflammation due to the released free fatty acids can ensue and a lymphocyte reaction may then transform the microcyst into a papule and then into a pustule with the gathering of pus.

It is accordingly apparent that any treatment directed to inhibiting the release of free fatty acids from sebum triglycerides, and arresting hyperkeratinisation of the follicular ostium would effect a regression of the primary symptoms of acne and would limit the development of new acneic lesions, particularly non-inflammatory lesions (comedones).

It has already been proposed in British Patent No. 1 388 836 (Medisan) published 26 March 1975, that certain organic esters, such as ethyl lactate, can be employed in the form of anhydrous alcoholic liquid solutions for the treatment of acne. According to this prior proposal, the absence of water in such compositions was considered essential in ensuring that the esters did not become prematurely hydrolysed, and hence ineffective as an acne treating material, until they had penetrated into the sebaceous glands and had dissolved in the sebum lipids. If premature hydrolysis of the esters occurred, it was apparent that skin penetration and dissolution in sebum lipids of the corresponding acid and alcohol resulting from hydrolysis would be impaired, because of their relatively low solubility compared with that of the ester. Hence, these hydrolysed esters were considered of little value in the topical treatment of acne.

It has also been proposed in British Patent No. 1 555 796 (Unilever) published 16 January 1980 that lower alkyl esters such as ethyl lactate can be employed in the form of aqueous alcoholic solutions for the treatment of acne. According to this prior proposal and contrary to the findings of Medisan as described in British Patent No.

1 388 836, it was found that ethyl lactate did not hydrolyse to a significant extent in compositions containing up to 50% by weight of water, provided that the pH value of the composition was below pH 7.

It has also been reported by Stotts et al in Journal of Investigative Dermatology, *69*, 219 (1977) which deals with the sensitisation of human skin to ethanol, that an aqueous alcoholic solution containing more than 40% by weight of ethanol is likely to cause eye irritation if applied to the face in the vicinity of the eyes. Furthermore, it is possible that excessive amounts of alcohol can stimulate sebum production which will lead to a worsening of the acneic condition.

It is apparent from these prior publications that the application to the acneic skin of the face of a liquid solution or suspension of an alkyl ester such as ethyl lactate containing a substantial quantity of a lower alkyl alcohol such as ethanol can present a hazard to the user, in that eye irritation can occur, unless the user is especially careful in the application of such a product to the skin of the face.

It has however now been shown that this problem can be overcome by applying the lower alkyl ester such as ethyl lactate to affected skin in the form of a solid stick which is anhydrous and substantially free from ethanol, thereby enabling the user to direct the ester only to the desired spot or area of affected skin without risking accidental application to sensitive areas such as the eyes and mouth. A pigment is also incorporated into the stick to enable the affected area of skin to be coloured to a suitable skin tone and any acne lesions masked, when the stick is applied to the skin.

Accordingly, the invention provides a substantially anhydrous, substantially ethanol-free cosmetic stick which comprises from 1 to 50% by weight of a $C_1$ to $C_4$ alkyl lactate or a mixture of said lactates, from 1 to 20% by weight of a pigment and from 5 to 98% by weight of solid matrix comprising a wax.

The invention also provides a process for making a cosmetic stick, which comprises distributing a $C_1$ to $C_4$ alkyl lactate or a mixture of said lactates in a mixture of matrix materials and shaping the mixture to form a solid stick.

The principal active compound in the composition according to the invention is one or more esters of $C_1$ to $C_4$ alkyl lactates, such as, for example, ethyl lactate, n-propyl lactate, iso-propyl lactate, n-butyl lactate, iso-butyl lactate and tert-butyl lactate. Mixtures of the $C_1$ to $C_4$ alkyl lactates can also be used.

The effectiveness of the foregoing class of esters is, as has already been stated, thought to be due to their ability to pass through the epidermis to reach the sebum in the sebaceous gland and pilosebaceous follicle, and there to dissolve in the sebum lipids where hydrolysis by lipases derived from bacterical contaminants will yield the corresponding acid and alcohol of these esters. The alcohol so formed is thought to exhibit antibacterial activity when formed *in situ* in the sebaceous glands and in this way the bacterial population, whose lipase activity otherwise contributes to acne formation following hydrolysis of sebum triglycerides with the release of free fatty acids, can thereby be reduced. Also the lactic acid which is formed *in situ* is capable of reducing the pH of the environment to a value below pH 6 thereby inhibiting bacterial lipase activity. Free lactic acid also appears to reduce keratinisation. The net result is that release from the sebum lipids of free fatty acids is reduced, and remission of the acne condition can be observed.

The preferred active ester is ethyl lactate.

The quantity of $C_1$ to $C_4$ alkyl lactate employed in the cosmetic stick forms from about 1% to about 50%, preferably from 5 to 25% by weight, based on the total weight of the stick in which it is employed. Cosmetic sticks containing less than 1% by weight of the alkyl lactate are likely to be ineffective for treating acne; cosmetic sticks containing more than 50% by weight of the alkyl lactate are unlikely to prove more effective in the treatment of this condition than those containing up to 50%.

The cosmetic stick also comprises a pigment to enable the skin to be coloured to a suitable tone or hue and any acne lesions or other skin blemishes masked when the stick is applied to the skin.

The pigment which is employed in the cosmetic stick of the invention can be any that is conventionally employed in the manufacture of make-up preparations intended for application to human skin.

Examples of pigments are natural pigments such as naturally occurring earth (aluminium silicates) whose colour depends mostly on the content of hydrated iron or manganese oxides, such as those known conventionally as yellow ochre, Terra di Sienna (brown), red bolus (brick red) and umber (dark brown). Such pigments can be heated to produce new shades, such as burnt Sienna.

Further examples of pigments are synthetic pigments such as synthetic iron oxides and synthetic ochres whose hues tend to be more intense than natural earth colours. Such synthetic pigments can be, for example, various yellow, brown-to-red and violet-shades. Synthetic pigments also include white pigments such as zinc oxide, titanium oxide and bismuth carbonate, whereas bismuth oxychloride can be used for producing pearly shades. Certain cobalt compounds can be used as blue synthetic pigments, such as cobalt green and ultramarine. Certain coal-tar dyes are also classified as synthetic pigments, an example of which is indanthrene blue.

Yet further examples of pigments are natural and synthetic lakes which are generally prepared by precipitating one or more water-soluble dyes on one or more insoluble substrates, and fixing them in such a way (usually by chemical reaction) that the end product becomes a colouring agent which is almost insoluble in water, oil or other solvents. An example of a natural lake is "Florentine Lake" which is obtained from a precipitation of carmine and brasilin (a red vegetable dye) on aluminium hydroxide. In a similar manner, a lake is prepared from alizarin red, a synthetic dye. The soluble dye or dyes used in the preparation of a lake determine its shade and intensity, the substrate and its covering effect. The most common substrates are zinc oxide, titanium oxide, aluminium hydroxide, aluminium phosphate, barium phosphate, barium sulphate, magnesium carbonate, alumina hydrates and kaolin.

Specific examples of pigments which are particularly preferred are those designated by the International Colour Index as follows:

12085: CI Pigment Red 4
15510: CI Acid Orange 7 (Orange II, a barium lake)
15585: CI Pigment Red 53
15630: CI Pigment Red 49 (a barium lake)
15850: CI Pigment Red 57 (a calcium lake)
15985: CI Food Yellow 3
19140: CI Food Yellow 4 (tartrazine)
45430: CI Food Red 14 (erythrosine)
73360: CI Vat Red 1
77491: CI Pigment Red 101 (Ferric oxide)
77492: CI Pigment Yellow 42 (hydrated ferric oxide)
77499: CI Pigment Brown 6 (ferroso-ferric hydroxide)

The pigment will normally be in the form of a finely divided powder, and is employed in the cosmetic stick of the invention at a level of from 1 to 20%, preferably from 5 to 15% by weight of the stick.

The cosmetic stick also comprises a solid matrix which serves as a diluent and vehicle for the alkyl lactate to enable the alkyl lactate to be applied precisely to the desired spot or area of affected skin at an appropriate concentration. The solid matrix also serves as a diluent and vehicle for the pigment to enable it to be effectively applied to the skin.

The solid matrix should be compatible with the lactate in that it does not promote significant hydrolysis of the lactate, while at the same time providing an attractive and useful stick which is durable in use, yet will release the alkyl lactate and pigment when positioned against the skin so that the user can apply the lactate and pigment with precision to the affected area.

The solid matrix comprises as an essential ingredient a wax or a waxy material having the properties of a wax, such as is conventionally employed in cosmetic stick manufacture.

Examples of such waxes or waxy materials which must be cosmetically acceptable are as follows:

microcrystalline wax, such as ceresin, carnauba wax, candelilla wax, beeswax, ozokerites or amorphous hydrocarbon waxes, paraffins, synthetic waxes, lacer buk wax, hydrogenated castor oil, spermaceti, cetyl alcohol and stearyl alcohol.

The amount of wax or waxy material to be employed as at least part of the solid matrix is from 5 to 98%, preferably from 10 to 50% and most preferably from 12 to 40% by weight of the cosmetic stick.

A cosmetic stick containing less than 5% by weight of a wax or waxy material generally does not exhibit satisfactory slip properties which enable it to be applied smoothly to the skin. On the other hand, a cosmetic stick which contains more than 98% by weight of wax will contain insufficient of the alkyl lactate to be effective in the treatment of acne.

The cosmetic stick can also optionally contain oils and fats such as are often employed in the manufacture of cosmetic sticks. Examples of such materials which must be cosmetically acceptable are as follows:

(i) oils and oily materials
vegetable oils, such as castor oil, mineral oils such as paraffin oil and vaseline oil, butyl stearate, isopropyl myristate, isopropylpalmitate, diethyl sebacate, diisopropyl adipate, cetyl lactate, hexadecyl stearate, cetyl oleate, oleyl alcohol, isostearyl alcohol, hexadecyl alcohol, wheatgerm oil, solvent oils, propylene glycol dicaprylate dicaprate, tetrahydrofurfuryl alcohol and acetate benzyl alcohol and phenylethyl alcohol.

(ii) fats and fatty materials
fats such as lard and tallow, saturated triglycerides, cocoa butter and cocoa butter substitutes, hydrogenated vegetable oils, petrolatum, lanolin, modified lanolin, such as hydrogenated lanolin, isopropyl lanolate, glycerol monostearate, acetoglycerides, lecithin, branched-chain hydrocarbons, alcohols and esters and long chain fatty acids such as stearic acid.

It is preferred to employ a suitable blend of waxes and oils and/or fats to obtain a stick with the required properties which will appeal to the consumer. Suitable blends are those conventionally employed in the art of lipstick and other make-up stick manufacture and as such are well known.

The total amounts of waxes and oils and/or fats which comprise the solid matrix will form from 5 to 98%, preferably from 10 to 80% by weight of the cosmetic stick.

The cosmetic stick should also preferably have a drop point of from 40° to 80°C, which property can be ensured by an appropriate choice of wax and fats and/or oils which can comprise the solid matrix. The choice of a suitable blend of these materials to achieve a particular drop point is a technique well known in the art of lipstick and eye make-up stick manufacture.

The Drop Point, also known as Melting Point can be determined by the method designated British Standards BS 894:1956 and BP (1958) Appl. IVa II pages 823 to 826.

The cosmetic sticks according to the invention are both substantially anhydrous and substantially free from ethyl alcohol and isopropyl alcohol.

The cosmetic sticks according to the invention can also contain other ingredients in addition to those already referred to, such as emollients, solvents, humectants, thickeners, moisturisers, antioxidants, emulsifiers, anti-inflammatory agents, healing agents, antiseptic, antibacterial, antibiotic and germicidal substances, keratolytic agents, abradant agents, and mineral charges, such as calcium carbonate, magnesium carbonate, titanium dioxide, silica and talc.

Examples of such other ingredients are to be found in McCutcheon's "Functional Materials" 1980 Annual published by M. C. Publishing Co., New Jersey.

Generally, the amount of each of the above other ingredients which can optionally be employed will be that recommended by the suppliers or manufacturers or that which is conventionally employed in the art, and which will not detrimentally affect the nature and function of the composition.

The cosmetic sticks according to the invention are prepared by a process which includes the steps of distributing a $C_1$ to $C_4$ alkyl lactate, or a mixture of said lactates, together with a pigment in a wax, and subsequently shaping the blend so obtained to form a solid stick.

According to a preferred process, the lactate or mixture of lactate, which includes ethyl lactate, is mixed together with other ingredients including the pigment, together with optional non-waxy non-oil ingredients such as germicide, antioxidant and perfume. Meanwhile the wax together with any oily or fatty ingredients which will comprise the solid matrix are melted without excessive heating and then cooled to a temperature not exceeding 80°C. The mixture including the lactate and pigment is then carefully added with stirring to the hot wax under reflux to prevent loss of the more volatile ingredients, and the blend while still in a molten state is poured into moulds. The hot helt in the moulds is allowed to cool slowly until solidification takes place and the individual cosmetic sticks are finally ejected from the mould and individually packed into applicator containers which provide an airtight seal to prevent premature loss of volatile ingredients, particularly the alkyl lactate.

The cosmetic sticks according to the invention are usually stored in a protected manner while not in use in a hand held stick container which can be a capped tube, preferably of the "push-up" or "wind-up" variety as conventionally employed for lipsticks.

The invention accordingly also provides a stick applicator comprising a cosmetic stick as herein defined contained in an airtight container from which the cosmetic stick can be exposed when required for application to the skin.

The invention is illustrated by the following Examples:

Example 1

A cosmetic stick was prepared from the following formulation:

| Alkyl lactate | % w/w |
|---|---|
| Ethyl lactate | 7.0 |

| Solid matrix | |
|---|---|
| Isostearyl alcohol | 20.0 |
| Saturated triglyceride | 18.5 |
| Isopropyl lanolate | 3.0 |
| Hydrogenated lanolin | 13.0 |
| Carnauba wax | 11.0 |
| Spermaceti (synthetic wax) | 5.0 |

| Other ingredients | |
|---|---|
| Pigment | 12.9 |
| Titanium dioxide | 8.6 |
| Antioxidant | 0.1 |
| Germicide | 0.1 |
| Perfume | 0.8 |

The above mixture in a heated state was filled into push-up stick holders and allowed to cool. The push-up stick so produced showed good stability and wear in use and its application to acneic spots on the affected faces of a panel of acne suffering volunteers showed remission of the acneic condition.

Example 2

A cosmetic stick was prepared from the following formulation:

| Alkyl lactate | % w/w |
|---|---|
| Ethyl lactate | 7.0 |

| Solid matrix | |
|---|---|
| Carnauba wax | 8.0 |
| Beeswax | 12.0 |
| Lanolin | 4.0 |
| Cetyl alcohol | 4.0 |
| Castor oil | 52.0 |

| Other ingredients | |
|---|---|
| Pigment | 10.0 |
| 2,4,4'-trichloro-2'-hydroxydiphenyl ether | 0.1 |
| Perfume, antioxidant | 2.9 |

The above mixture is a heated state was filled into lipstick-type containers and allowed to cool.

Example 3

A cosmetic stick according to the invention can be prepared from the following ingredients:

| Alkyl lactate | % w/w |
|---|---|
| ethyl lactate | 10 |

| Solid greasy matrix | |
|---|---|
| vaseline oil | 22.5 |
| lacer buk wax | 6.5 |
| castor oil | 7.5 |
| paraffin oil | 5.0 |
| petrolatum | 24.0 |

| Other ingredients | |
|---|---|
| antioxidant | 0.03 |
| magnesium carbonate | 4.67 |
| perfume | 0.5 |
| pigment | 1.0 |
| emollient | 18.3 |
| | 100.00 |

Example 4

A cosmetic make-up stick for acne treatment according to the invention can be prepared from the following ingredients:

| Alkyl lactate | % w/w |
|---|---|
| ethyl lactate | 15.0 |

| Solid greasy matrix | |
|---|---|
| Ozokerite white | 6.0 |
| Carnauba wax | 4.0 |
| Candelilla wax | 8.0 |
| Propylene glycol dicaprylate dicaprate | 46.5 |

| Other ingredients | |
|---|---|
| Preservative | 0.2 |
| Montmorillonite clay | 10.0 |
| Pigment mix | 10.0 |
| Perfume | 0.3 |
| | 100.0 |

Example 5

Example 1 can be repeated by substituting n-propyl lactate for ethyl lactate.

Example 6

Example 1 can be repeated by substituting n-butyl lactate for ethyl lactate.

Example 7

Example 1 can be repeated by substituting a mixture in equal proportions of iso-propyl lactate and iso-butyl lactate for ethyl lactate.

**Claims**

1. A substantially anhydrous, ethanol-free cosmetic stick, having a Melting Point of from 40° to 80°C comprises:

i) from 1 to 50% by weight of a $C_1$ to $C_4$ alkyl lactate or a mixture of said lactates; and
ii) from 1 to 20% by weight of pigment; and
iii) from 5 to 98% by weight of a solid matrix comprising a wax.

2. A cosmetic stick according to claim 1, in which the alkyl lactate is chosen from ethyl lactate, n-propyl lactate, iso-propyl lactate, n-butyl lactate, iso-butyl lactate, tert-butyl lactate and mixtures thereof.

3. A cosmetic stick according to claim 1, 2 or 3, in which the alkyl lactate forms from 5 to 25% by weight of the stick.

4. A cosmetic stick according to claim 1, 2 or 3, in which the pigment forms from 5 to 15% by weight of the stick.

5. A cosmetic stick according to any preceding claim, in which the wax forms from 10 to 50% by weight of the stick.

6. A cosmetic stick according to claim 5, in which the wax forms from 12 to 40% by weight of the stick.

7. A cosmetic stick according to any preceding claim, in which the solid matrix additionally comprises an oil and/or an oily material.

8. A cosmetic stick according to any preceding claim, in which the solid matrix additionally comprises a fat and/or a fatty material.

9. A cosmetic stick according to any preceding claim, which additionally comprises a healing agent, an antiseptic agent, an antibacterial agent and antibiotic or a germicidal agent or a mixture thereof.

10. A process for making a cosmetic stick according to any preceding claim, which process comprises distributing a $C_1$ to $C_4$ alkyl lactate, or a mixture of said lactates, together with a pigment in a wax, and shaping the mixture to form a solid stick.

11. A hand held applicator containing a cosmetic stick according to any of claims 1 to 9.

**Revendications**

1. Un bâtonnet cosmétique pratiquement anhydre, exempt d'éthanol, possédant un point de fusion de 40° à 80°C comprend:

i) 1 à 50% en poids d'un lactate d'alkyle en $C_1$ à $C_4$ ou un mélange desdits lactates;
ii) 1 à 20% en poids de pigment; et
iii) 5 à 98% en poids d'une matrice solide contenant une cire.

2. Bâtonnet cosmétique selon la revendication 1, dans lequel le lactate d'alkyle est choisi parmi le lactate d'éthyle, le lactate de n-propyle, le lactate d'isopropyle, le lactate de n-butyl, le lactate d'isobutyle, le lactate de tert. butyle et leurs mélanges.

3. Bâtonnet cosmétique selon la revendication 1 ou 2, dans lequel le lactate d'alkyle représente 5 à 25% en poids du bâtonnet.

4. Bâtonnet cosmétique selon la revendication 1, 2 ou 3, dans lequel le pigment représente 5 à 15% en poids du bâtonnet.

5. Bâtonnet cosmétique selon l'une quelconque des revendications précédentes, dans lequel la cire représente 10 à 50% en poids du bâtonnet.

6. Bâtonnet cosmétique selon la revendication 5, dans lequel la cire représente 12 à 40% en poids du bâtonnet.

7. Bâtonnet cosmétique selon l'une quelconque des revendications précédentes, dans lequel la matrice solide contient en outre une huile et/ou une matière huileuse.

8. Bâtonnet cosmétique selon l'une quelconque des revendications précédentes, dans lequel la matrice solid contient en outre une graisse et/ou une matière grasse.

9. Bâtonnet cosmétique selon l'une quelconque des revendications précédentes contenant en outre un cicatrisant, un agent antiseptique, un agent antibactérien et un agent antibiotique ou germicide ou un mélange de ceux-ci.

10. Un procédé de fabrication d'un bâtonnet cosmétique selon l'une quelconque des revendications précédentes, qui consiste à répartir un lactate d'alkyle en $C_1$ à $C_4$ ou un mélange desdits lactates dans une cire, conjointement avec un pigment et à façonner le mélange pour former un bâtonnet solide.

11. Un applicateur à main contenant un bâtonnet cosmétique selon l'une quelconque des revendications 1 à 9.

**Patentansprüche**

1. Ein im wesentlichen wasserfreier, ethanolfreier kosmetischer Stift mit einem Schmelzpunkt von 40 bis 80°C umfaßt:

i) 1 bis 50 Gew.-% eines $C_1$- bis $C_4$-Alkyllactats oder eines Gemischs der Lactate und
ii) 1 bis 20 Gew.-% Pigment und
(iii) 5 bis 98 Gew.-% einer festen, ein Wachs umfassenden Matrix.

2. Kosmetischer Stift nach Anspruch 1, in dem das Alkyllactat unter Ethyllactat, n-Propyllactat, Isopropyllactat, n-Butyllactat, Isobutyllactat, tert.-Butyllactat und deren Gemischen ausgewählt ist.

3. Kosmetischer Stift nach Anspruch 1, 2 oder 3, in dem das Alkyllactat 5 bis 25 Gew.-% des Stiftes bildet.

4. Kosmetischer Stift nach Anspruch 1, 2 oder 3, in dem das Pigment 5 bis 15 Gew.-% des Stiftes bildet.

5. Kosmetischer Stift nach irgend einem vorhergehenden Anspruch, in dem das Wachs 10 bis 50 Gew.-% des Stiftes bildet.

6. Kosmetischer Stift nach Anspruch 5, in dem das Wachs 12 bis 40 Gew.-% des Stiftes bildet.

7. Kosmetsicher Stift nach irgend einem vorhergehenden Anspruch, in dem die feste Matrix zusätzlich ein Öl und/oder ein öliges Material umfaßt.

8. Kosmetischer Stift nach irgend einem vorhergehenden Anspruch, in dem die feste Matrix außerdem ein Fett und/oder ein fettiges Material umfaßt.

9. Kosmetischer Stift nach irgend einem vorhergehenden Anspruch, der außerdem ein heilendes Mittel, ein antiseptisches Mittel, ein antibakterielles Mittel und antibiotisches oder ein keimtötendes Mittel oder ein Gemisch hiervon umfaßt.

10. Verfahren zur Herstellung eines kosmetischen Stiftes gemäß irgend einem vorhergehenden Anspruch, das das Verteilen eines $C_1$—$C_4$-Alkyllactats oder eines Gemischs der Lactate, zusammen mit einem Pigment in einem Wachs und das Formen des Gemischs zur Bildung eines festen Stiftes umfaßt.

11. Ein von Hand gehaltener Applikator, enthaltend einen kosmetischen Stift gemäß irgend einem der Ansprüche 1 bis 9.